# EUROPEAN PATENT SPECIFICATION

(11) **EP 1 874 806 B1**
(45) Date of publication and mention of the grant of the patent: **17.02.2010**
(21) Application number: 06726753.4
(22) Date of filing: 13.04.2006
(51) Int. Cl.: C07K 14/21, C07K 14/32, A61K 39/07, A61K 39/104, C07K 16/12, A61P 37/04, A61P 31/04

(54) **VACCINE AGAINST BURKHOLDERIA INFECTIONS**
IMPFSTOFF GEGEN BURKHOLDERIA-INFEKTIONEN
VACCIN CONTRE LES INFECTIONS BURKHOLDERIA

(30) Priority: 15.04.2005 GB 0507632
(43) Date of publication of application: 09.01.2008
(73) Proprietor: THE SECRETARY OF STATE FOR DEFENCE, Salisbury Wiltshire SP4 0JQ (GB)
(72) Inventor: HARLAND, David Neil, Belper, Derbyshire DE56 0EW (GB); ATKINS, Helen Susan, Salisbury, Wiltshire SP4 0JQ (GB); WALKER, Nicola Jane, Salisbury, Wiltshire SP4 0JQ (GB); NELSON, Michelle, Salisbury, Wiltshire SP4 0JQ (GB); ATKINS, Timothy Phillip, Salisbury, Wiltshire SP4 0JQ (GB); TITBALL, Richard William, Salisbury, Wiltshire SP4 0JQ (GB)
(74) Representative: Farnsworth, Alastair Graham
(86) International application number: PCT/GB2006/001354
(87) International publication number: WO 2006/109071

(56) References cited:
- WOO P C ET AL: "Cloning and characterisation of malE in Burkholderia pseudomallei." JOURNAL OF MEDICAL MICROBIOLOGY. APR 2001, vol. 50, no. 4, April 2001 (2001-04), pages 330-338, XP002399893 ISSN: 0022-2615
- HOLDEN MATTHEW T G ET AL: "Genomic plasticity of the causative agent of melioidosis, Burkholderia pseudomallei." PROCEEDINGS OF THE NATIONAL ACADEMY OF SCIENCES OF THE UNITED STATES OF AMERICA. 28 SEP 2004, vol. 101, no. 39, 28 September 2004 (2004-09-28), pages 14240-14245, XP002399894 ISSN: 0027-8424 cited in the application -& DATABASE EMBL [Online] 16 September 2004 (2004-09-16), "Burkholderia pseudomallei strain K96243, chromosome 1, complete sequence." XP002399907 retrieved from EBI accession no. EM_PRO:BX571965 Database accession no. BX571965 -& DATABASE EMBL 16 September 2004 (2004-09-16), "Burkholderia pseudomallei strain K96243, chromosome 2, complete sequence" XP002399908 retrieved from EBI accession no. EM_PRO:BX571966 Database accession no. BX571966 -& DATABASE UniProt 25 October 2004 (2004-10-25), "Putative lipoprotein releasing system transmembrane protein" XP002399909 ret
- GARMORY HELEN S ET AL: "ATP-binding cassette transporters are targets for the development of antibacterial vaccines and therapies." INFECTION AND IMMUNITY. DEC 2004, vol. 72, no. 12, December 2004 (2004-12), pages 6757-6763, XP002399895 ISSN: 0019-9567 cited in the application
- ATKINS HELEN S ET AL: "The identification and evaluation of ATP binding cassette systems in the intracellular bacterium Francisella tularensis." RESEARCH IN MICROBIOLOGY. 2006 JUL-AUG, vol. 157, no. 6, July 2006 (2006-07), pages 593-604, XP002399896 ISSN: 0923-2508

## Description

This invention relates to ATP-binding cassette (ABC) system proteins derived from *Burkholderia pseudomallei,* to their use in producing a protective immune response in mammals and to the preparation of vaccines against melioidosis and glanders. The invention also provides a method for the production of such proteins.

*Burkholderia pseudomallei* is a saprophytic, Gram negative, non-spore forming bacillus most commonly isolated in South East Asia. In this region and particularly in Thailand, Vietnam and Northern Australia, *B. pseudomallei* is a major cause of morbidity and mortality as the causative agent of the emerging infection melioidosis. Melioidosis has four different forms of disease: acute, sub-acute, chronic and sub-clinical. The acute, sub acute and chronic infections progress systemically giving rise to septicaemic disease, which in untreated cases has a mortality rate of 80-90% with death occurring within 24-48 hours of the onset of symptoms. Acute septicaemia is usually associated with patients with an existing immunodeficiency and in many cases adult melioidosis is the result of re-infection or re-activation of latent infections, usually contracted in early in life in endemic areas. There is currently no licensed vaccine for melioidosis. The primary treatment regime for melioidosis consists of antibiotic treatment with intravenous ceftazidime for 10-14 days. However, *B. pseudomallei* is amongst the growing number of bacterial pathogens that exhibit an increasingly high level of antibiotic resistance and as a result the need for an effective vaccine is pressing. To date, no proteins have been identified as protective against melioidosis.

ABC systems are responsible for the transport of a wide variety of different molecules across cellular membranes. The ABC systems constitute one of the largest protein superfamilies and are ubiquitous in nature, being found in bacteria, eukarya and archea (C.F. Higgins, Annu. Rev. Cell Biol. 8 (1992) 67-113). Bacterial ABC transporters are extremely versatile transport systems which vary greatly in size and function to either import or export a range of substances denoted as 'allocrites'. Due to the diverse nature of the allocrites, these transport systems have been shown to play different roles in many cellular functions including bacterial metabolism and virulence. For example, importers are often involved in the uptake of nutrients such as sugars, iron and peptides, and are therefore important in bacterial metabolism. Furthermore, inactivation of ABC import systems can lead to attenuation. For example, inactivation of the *sfbABC* iron uptake system in *Salmonella enteritidis* results in attenuation in mice. In comparison, ABC transporters that function to export allocrites are often more directly associated with virulence and pathogenicity, either directly through the export of toxins such as haemolysin or by improving survival through antibiotic resistance.

The common feature of ABC systems is the presence of protein components containing highly conserved ATP-binding cassettes (ABCs) that are responsible for the generation of energy for the transport of allocrites through the hydrolysis of ATP to ADP. ABCs include Walker A and Walker B sites for binding ATP, a number of membrane spanning domains (MSDs), and a specific signature sequence. The normal structural arrangement within ABC proteins is a four domain arrangement - two domains containing ABCs and two domains containing MSDs arranged on one or more polypeptide chains. Other proteins may also be involved in import and export of allocrites in ABC systems. For example, periplasmic solute binding proteins are usually involved in import in Gram negative organisms. Additional outer membrane proteins such as TolC may be required for the export of allocrites.

A number of ABC system proteins have been identified as immunogenic, including predicted solute binding proteins and ABC containing proteins, usually identified through a process of screening anti-serum raised against whole bacterial cells. This identification of immunogenic ABC system proteins, coupled with additional evidence of the ability of ABC system proteins to protect against *Brucella abortus" Mycobacterium tuberculosis* and *Streptococcus pneumoniae* infection, has prompted the assertion that ABC system proteins may constitute potential vaccine antigens (see, for example, Garmory, R.S. & Titball, R.W., Infection and Immunity, 72 (2004), 6757-6763). However, despite the fact that this has led to the targeting of such proteins as potential vaccine antigens, it has been found that many, if not most, of the ABC system proteins isolated to date are largely ineffective as vaccine antigens, irrespective of their ability to elicit an immune response (see, for example, Atkins, H.S. et al, Research in Microbiology 157 (2006), 593-604). Certainly, there is no indication in the prior art that ABC system proteins, in particular those derived from *Burkholderia* species, may be used as protective antigens.

The applicants have now identified several proteins which are derived from the ABC systems of *Burkholderia pseudomallei* and which produce a protective immunogenic response against *Burkholderia* infection, when administered to a mammal. The proteins have been cloned, expressed and purified and their potential to induce protective immunity against *Burkholderia pseudomallei* tested in the mouse model of infection. Consequently, these proteins are useful as vaccines against diseases caused by *Burkholderia* infections, such as Melioidosis and Glanders.

Specifically, these proteins are derived from ABC systems of *Burkholderia pseudomallei* and include proteins which are predicted to be present in the region of the periplasmic space and outer membrane areas of the bacterium, such as periplasmic binding proteins, and also inner membrane proteins which are thought to be involved in export processes. The proteins are capable of producing a protective immune response against *Burkholderia pseudomallei* and *Burkholderia mallei* and, as such, fragments of the proteins which are capable of providing a protective immune response and variants of the proteins which are capable of providing a protective immune response are also included within the scope of the invention.

As used herein, the term "protein" means a sequence of amino acids joined together by peptide bonds. The amino acid sequence of the protein is determined by the sequence of DNA bases which encode the amino acids of the protein. As used herein protein includes polypeptides and herein, the terms "protein" and "polypeptide" are used interchangeably.

According to the present invention there is provided a protein expressed by an ABC system gene of *Burkholderia pseudomallei* selected from the group consisting of OppA, PotF and LolC, or a fragment or a variant of said protein, wherein the protein, fragment or variant is capable of producing a protective immune response against *Burkholderia mallei* or *Burkholderia pseudomallei* infection, for use as a medicament.

In particular, the protein is for use as a prophylactic or therapeutic vaccine against infection by *Burkholderia mallei* and *Burkholderia pseudomallei.* Prophylactic vaccines are particularly preferred.

In the context of the present invention the expression "variant" as used herein refers to sequences of amino acids which differ from the base sequence from which they are derived in that one or more amino acids within the sequence are substituted for other amino acids. Amino acid substitutions may be regarded as "conservative" where an amino acid is replaced with a different amino acid with broadly similar properties. "Non-conservative" substitutions are where amino acids are replaced with amino acids of a different type. Broadly speaking, fewer non-conservative substitutions will be possible without altering the biological activity of the polypeptide. Suitably variants will be at least 80% identical, preferably at least 90% identical, and more preferably at least 95% identical to the base sequence.

Identity in this instance can be judged, for example, using the BLAST program (version 2.2.12) found at http:www.ncbi.nlm.nih.gov/BLAST or by using the algorithm of Lipman-Pearson, with Ktuple:2, gap penalty:4, Gap Length Penalty:12, standard PAM scoring matrix (Lipman, D.J. and Pearson W.R., Rapid and Sensitive Protein Similarity Searches, Science, 1985, vol.227, 1435-1441).

The term "fragment" refers to any portion of the given amino acid sequence of a polypeptide which has the same activity as the complete amino acid sequence. Fragments will suitably comprise at least 5 and preferably at least 10 consecutive amino acids from the basic sequence, or combinations of these fragments. In order to retain activity, fragments will suitably comprise at least one epitopic region. Fragments comprising epitopic regions may suitably be fused together to form variants within the above definition.

The phrase "producing a protective immune response" as used herein means that the substance is capable of generating a protective immune response in a host organism, such as a mammal, for example, a human, to whom it is administered.

Proteins of the invention are **characterised in that** they bind directly to, or are components of systems that bind to adenosine triphosphate (ATP), for example, periplasmic binding proteins such as OppA (CDS no. YP 112142) and PotF (CDS no. YP 106734), and inner membrane export/releasing proteins such as "LolC" obtained from *Burkholderia pseudomallei.* Further examples of such proteins include fragments or variants of OppA, PotF or LolC as described above.

In particular it has been found that polypeptides with any of SEQ ID Nos 4, 5 or 6 are preferred examples of proteins of the invention. A more preferred example is the polypeptide with SEQ ID no4. Therefore this protein and protective fragments and variants thereof form a particularly preferred embodiment of the invention.

Proteins as described above may be prepared using methods according to another aspect of the invention. Such methods comprise firstly, identifying the ABC system genes in the genome of the *Burkholderia* species, for example the *Burkholderia pseudomallei* K96243 genome sequence. ABC system genes may be identified by scanning the genome for ABC system signature sequences, such as PS00211, using any suitable commercially available genome viewing software, such as Artemis. Once the ABC system proteins have been identified, the genes that encode the proteins, or the fragments of the genes that encode specific peptide regions of the corresponding ABC system proteins are amplified. The amplified gene fragments may then be cloned into an expression vector to produce a recombinant plasmid, which may, in tum be transformed into a host cell. Host cells may be prokaryotic or eukaryotic cells. It is preferred that the host cell is a prokaryotic cell, such as *Escherichia coli.*

According to a second aspect of the invention there is provided a pharmaceutical composition comprising a protein expressed by an ABC system gene of *Burkholderia pseudomallei,* selected from the group consisting of of OppA, PotF and LolC, or a fragment or a variant of said protein, wherein the protein, fragment, or variant is capable of producing a protective immunogenic response against *Burkholderia mallei* or *Burkholderia pseudomallei,* in combination with a pharmaceutically acceptable carrier or excipient.

Suitable excipients and carriers will be known to those skilled in the art. These may include solid or liquid carriers. Suitable liquid carriers include water or saline. The proteins of the composition may be formulated into an emulsion or alternatively they may be formulated in, or together with, biodegradable microspheres or liposomes.

Suitably the composition further comprises an adjuvant which stimulates the host's immune response. Particularly suitable adjuvants include Alhydrogel^{™}, MPL+TDM^{™} and Freunds Incomplete Adjuvant.

In a preferred embodiment, the pharmaceutical composition further comprises an additional immunogenic polypeptide derived from *Burkholderia pseudomallei.* The additional polypeptide may be another selected from the group of proteins described herein or may be another protective antigen for *Burkholderia pseudomallei.*

In accordance with a particular aspect of the invention, there is provided a nucleic acid which encodes a protein as described above. Particular examples include nucleic acids which encode the proteins of SEQ ID NO 4, 5 or 6. A nucleic acid which encodes protein of SEQ ID no 4 is preferred. Particular examples of other preferred nucleic acids are those of SEQ ID nos 1, 2 and 3 and, in particular, the nucleic acid of SEQ ID no 3 is more preferred.

Such nucleic acids can be used to produce the proteins described herein for use as medicaments. For instance they may be incorporated into an expression vector, which is used to transform an expression host, such as a prokaryotic or eukaryotic cell and, in particular, a prokaryotic cell such as *Escherichia coli,* using recombinant DNA technology as is well understood in the art. Such vectors, cells and expression methods form further aspects of the invention.

Alternatively, the nucleic acids can be used in "live" or "DNA" vaccines to deliver the protein to the host animal.

Thus, in a further aspect of the invention there is provided a nucleic acid, as described above for use as a medicament.

When used in this way, the nucleic acids will suitably be included within an expression vector such as a plasmid, and incorporated into a pharmaceutical composition. Thus, yet a further aspect comprises a pharmaceutical composition comprising a nucleic acid as described above in combination with a pharmaceutically acceptable carrier.

Other carriers that are suitable for use in the immunogenic composition include vectors for the delivery of the polypeptide, such as viral vectors (such as vaccinia or adenovirus), bacterial vectors (such as *Salmonella*) or plasmids. These vectors will allow expression of the polypeptide encoded by the nucleic acid within the host animal.

Suitable viral or bacterial vectors advantageously comprise human or animal gut colonising organisms that have been transformed using recombinant DNA to enable them to express the polypeptide or a protective epitopic part of the polypeptide. *Salmonella*-based vectors are particularly suitable but other live vectors are known in the art. Alternatively the composition may include so called naked DNA vaccines, wherein the nucleic acid such as DNA which encodes the required polypeptide is included in a plasmid.

The composition of the present invention may be used as vaccine against infections caused by *Burkholderia* species, such as melioidosis and glanders. The vaccine may be administered prophylactically to those at risk of exposure to *Burkholderia mallei or Burkholderia pseudomallei* or may be administered as a therapeutic treatment to persons who have already been exposed to these pathogens.

The route of administration of the vaccine may be varied depending on the formulation of the polypeptides of the composition. The composition may be suited to parenteral administration (including intramuscular, subcutaneous, intradermal, intraperitoneal and intravenous administration) but may also be formulated for non-parenteral administraion (including intranasal, inhalation, oral, buccal, epidermal, transcutaneous, ocular-topical, vaginal, rectal administration).

In another aspect, the invention provides the use of a protein as described above in the preparation of a medicament for prophylactic or therapeutic vaccination against *Burkholderia mallei* or *Burkholderia pseudomallei.*

According to a further aspect the present invention relates to an antibody raised against the polypeptides described above, or a binding fragment thereof. Since the polypeptides are immunogenic, they are capable of inducing an immune response in a mammal to which they are administered. Antibodies may be raised in-vivo against the complete polypeptides, or they may be raised against suitable epitopic fragments of the polypeptides using conventional methods.

Binding fragments include Fab, F(ab')2, Fc and Fc'.

Antibodies may be polyclonal or monoclonal. Hybridoma cell lines which generate monoclonal antibodies of this type form a further aspect of the invention.

Antibodies themselves, for example in the form of sera comprising such antibodies may be useful in the passive vaccination and/or treatment of compromised individuals.

Accordingly, aspects of the invention provide a method of protecting a human or animal body from the effects of infection with *Burkholderia mallei* or *Burkholderia pseudomallei* comprising administering to the body a vaccine comprising a polypeptide or a pharmaceutical composition as described above. The polypeptide is capable of inducing a protective immune response in a mammal to which it is administered and the ability to elicit an effective immune response may be provided by an epitopic fragment or a variant of said protein. Particular examples of suitable proteins include those described above. It is preferred that the protein is LolC (SEQ ID no.4) or a protective fragment or a protective variant thereof.

The polypeptide may be administered to the body by means of a "live" or DNA vaccine as described above.

The polypeptide utilised in the above method (and also present in the composition as described above) may be isolated from a suitable subspecies of *Burkholderia pseudomallei,* such as *Burkholderia pseudomallei* strain K96243 or, alternatively, it may be expressed in recombinant form and purified as appropriate as described as described in the examples herein.

The invention will now be particularly described by way of example with reference to the accompanying diagrammatic drawings where:
Figure 1 shows the IgG1 and IgG2a response in mice to immunisation with PotF, LolC and OppA proteins and also the response with DNA vaccines prepared by transformation of the genes encoding said proteins.
Figure 2 shows survival curves for groups of six mice immunised with purified recombinant LolC, Oppa and PotF, control samples and naive mice which were subsequently challenged with approximately 4 x 10⁴ CFU of *Burkholderia pseudomallei* K96243.
Figure 3 shows survival curves for groups of six mice immunised with DNA vaccines encoding LolC, Oppa and PotF, control samples and naive mice which were subsequently challenged with approximately 4 x 10⁴ CFU of *Burkholderia pseudomallei* K96243.

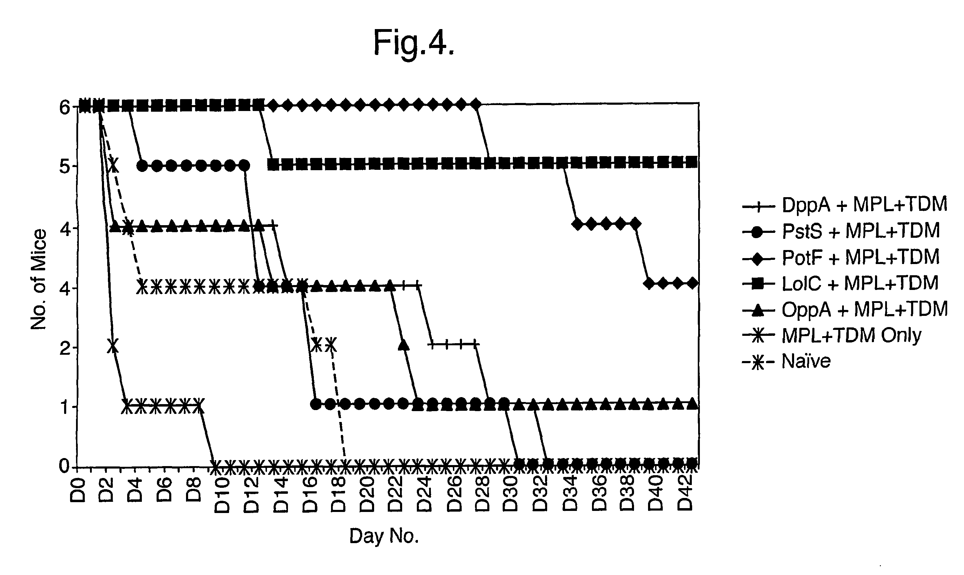

Figure 4 shows survival curves for groups of 6 mice immunised with purified recombinant ABC system proteins (DppA, PstS, PotF, LolC, OppA) together with MPL+TDM adjuvant and naive mice that were subsequently challenged with approximately 4 x 10⁴ CFU of *Burkholderia pseudomallei* K96243

### Bacterial strains, media and culture conditions

*Escherichia coli* TOP10F' cells (Invitrogen Ltd. Paisley, UK) were used for cloning experiments and *E. coli* BL21 (DE3) pLysS cells (Invitrogen Ltd. Paisley, UK) were used for protein expression studies. The *Burkholderia* spp. strains from which purified DNA was provided are listed in Table 1. *Burkholderia pseudomallei* K96243 was used to challenge all mice. *E. coli* strains were cultured in Luria broth (L-broth) shaking at 180 rpm or on Luria agar (L-agar) plates at ACDP containment level 2 conditions. L-broth consisted of 1% (w/v) Bacto tryptone, 0.5% (w/v) Bacto yeast extract, and 0.5% (w/v) sodium chloride in distilled water. L-agar was prepared by the addition of 2% (w/v) Bacto agar to L-broth. Luria media was supplemented with ampicillin and/or chloramphenicol when appropriate. *B. pseudomallei* strains were cultured on nutrient agar or statically in nutrient broth at ACDP containment level 3 conditions. All bacteria were cultured at 37°C for 18 hours unless otherwise stated.

**Table 1. The presence of genes encoding ABC system proteins in Burkholderia strains.**

| *Burkholderia* strain | Gene | | |
|---|---|---|---|
| | *oppA* | *potF* | *lolC* |
| *B. pseudomallei* E38 | ✔ | ✔ | ✔ |
| *B. pseudomallei 44* | ✔ | ✔ | ✔ |
| *B. pseudomallei* 2889 | ✔ | ✔ | ✔ |
| *B. pseudomallei* Mal6 | ✔ | ✔ | ✔ |
| *B. pseudomallei* NCTC 4845 | ✔ | ✔ | ✔ |
| *B. pseudomallei* 708A | ✔ | ✔ | ✔ |
| *B. pseudomallei* 576 | ✔ | ✔ | ✔ |
| *B. pseudomallei* 204 | ✔ | ✔ | ✔ |
| *B. pseudomallei* E8 | ✔ | ✔ | ✔ |
| *B. pseudomallei* K96243 | ✔ | ✔ | ✔ |
| *B. mallei ATCC* 10247 | ✔ | ✔ | ✔ |
| *B. thailandensis* E82 | ✔ | ✔ | ✔ |
| *B. thailandensis* E27 | ✔ | ✔ | ✔ |

| | | | |
|---|---|---|---|
| ✔ = present | | | |

### Identification and selection of potential antigens

The Artemis genome viewer was used to locate PS00211, the ABC systems signature sequence, in the genome sequence of *B. pseudomallei* K96243 sequence (Holden, M.T.G. et al Proc. Nat. Acad. Sci. USA 101 (2004) 14240-14245). BLASTP searches were performed on the translated genes containing the signature sequence and on neighbouring genes in order to identify all ABC system genes in the *B. pseudomallei* K96243 sequence. ABC system proteins considered potentially involved in the virulence or immunogenicity of *B. pseudomallei* were selected for further study.

### Screening of different Burkholderia sp. strains for genes encoding selected proteins

Oligonucleotide primer pairs were designed to amplify internal fragments of the genes encoding the selected proteins. Genomic DNA prepared from different *Burkholderia* sp. strains was described previously (Table 1). PCR was performed using oligonucleotide primers and conditions detailed in Table 2. Amplified DNA was visualised by agarose gel electrophoresis to determine presence of genes encoding selected proteins in the different *Burkholderia* strains.

**Table 2. Oligonucleotide primers and PCR conditions for the amplification of internal fragments of oppA, potF and lolC for screening Burkholderia strains**

| Gene | PCR Primers | PCR Conditions | | | | |
|---|---|---|---|---|---|---|
| | | Den. ºC^{a} | Den. ºC | Ann. ºC^{b} | Ext. ºC^{c} | Ext. ºC |
| *oppA* | 5'-CGGCCGCGGATTTCGTAT-3' F^{d} | 95 | 95 | 50 | 72 | 72 |
| | 5'-GCAGCGCGGTCTCGTCGTTCT-3' R^{e} | | | | | |
| *potF* | 5'-ATCCAGGCCGGCATCTTCA-3' F 5'-AGCGCGGCGTCCTTGTTC-3' R | 96 | 96 | 66 | 72 | 72 |
| *lolC* | 5'- AACTTTGAGGGCATGCTTACCGTC- 3' F | 94 | 94 | 50 | 72 | 72 |
| | 5'- TCATTCATAGCGCACCGCCTCCGC -3' R | | | | | |

| | | | | | | |
|---|---|---|---|---|---|---|
| ^{a} = Denaturing temperature ^{b} = Annealing temperature ^{c} = Extension temperature ^{d} = Forward primer ^{e} = Reverse primer | | | | | | |

### Cloning and expression

Amino acid sequences of the selected proteins were analysed using TMHMM v 2.0 (http://www.cbs.dtu.dk/services/TMHMM-2.0/) and SignalP (http://www.cbs.dtu.dk/services/SignalP/) to identify regions encoding transmembrane domains and signal peptides respectively. Oligonucleotide primers were designed to amplify DNA sequence encoding the non-membrane/non-signal peptide regions of the proteins. These gene truncates were amplified from *B. pseudomallei* K96243 genomic DNA by PCR using oligonucleotide primers and conditions listed in Table 3. The amplified gene fragments were cloned into the pCR^{®}T7/NT-TOPO expression vector (Invitrogen Ltd. Paisley, UK), and the recombinant plasmids were transformed into *Escherichia coli* TOP10F' cells (Invitrogen Ltd. Paisley, UK), according to manufacturer's instructions. Sequencing reactions were performed by MWG Biotech (Milton Keynes, UK) to ensure correct cloning and the incorporation of a N-terminal His₆-tag. Subsequently, *E. coli* BL21 (DE3) pLysS cells (Invitrogen Ltd. Paisley, UK) were transformed with the recombinant plasmids, according to manufacturer's instructions. Transformants were grown in culture and the addition of 1 mM Isopropyl-β-D-thiogalactoside (IPTG) was used to induce the expression of protein from the recombinant plasmids. Protein expression was confirmed by SDS-PAGE performed using Phastsystem gels and apparatus (Amersham Biosciences Chalfont-St Giles, UK) and visualised by PhastGel Blue R (Amersham Biosciences Chalfont-St Giles, UK). Expressed proteins were transferred to a membrane for detection by Western blot using transfer apparatus of the Phastsystem with the proteins detected with anti-His antibody and developed using 3,3'-Daminobenzidine (DAB) (Sigma-Aldrich Co. Ltd., Poole, UK).

**Table 3. Oligonucleotide primers and PCR conditions for the amplification of non-signal peptide/non-membrane regions of oppA, potF and lolC for cloning experiments**

| **Gene** | PCR Primers | PCR Conditions | | | | |
|---|---|---|---|---|---|---|
| | | Den. ºC^{a} | Den. ºC | Ann. ºC^{b} | Ext. ºC^{c} | Ext. ºC |
| *oppA* | 5'-AGGATCCTAATGTCGAATGTCACGCTC-'3 F BamHI | 96 | 96 | 61 | 72 | 72 |
| | 5'-AAAGCTTTATTCAGTGCTTGATCAGGT-3' R HindIII | | | | | |
| *potF* | 5'-AGAATTCTAATGAAGGATACGCAGCTC-'3 F EcoRI | 96 | 96 | 49 | 72 | 72 |
| | 5'- AGCGGCCGCTATTCAGCGTCCCGATTTCAG- '3 R NotI | | | | | |
| *lolC* | 5'-AGGATCCTAATGGTGCTGTAGGTGATG-'3 F | 96 | 96 | 55 | 72 | 72 |
| | BamHI 5'-AAAGCTTTATTCACCGCTTCTCGATCT-'3 R HindIII | | | | | |

### Protein purification

To produce purified protein, *E. coli* BL21 (DE3) pLysS cells expressing recombinant protein were centrifuged at 10,000 rpm for 15 min. Pelleted cells were resuspended in Phosphate buffered saline (PBS) plus 300 µg ml⁻¹ DNAase I and Complete EDTA-free protease inhibitor (Roche Diagnostics Ltd Lewes, UK). Cell suspensions were sonicated at 10 microns in 3 x 30 sec pulses and lysed cells were centrifuged at 15,000 rpm for 20 min. The supernatants were removed and sterilised through a 0.22 µm filter. Immobilised Metal Affinity Chromatography (IMAC) was carried out under the control of AKTA Fast Protein Liquid Chromatography (FPLC) (Amersham Biosciences Chalfont-St Giles, UK) and using Unicorn software version 4.0 (Amersham Biosciences Chalfont-St Giles, UK). Filtered supernatant was applied to a 1 ml His-Trap column (Amersham Biosciences Chalfont-St Giles, UK) with Start buffer (50mM tris, 750 mM NaCl pH 7.5) and wash fractions were collected, according to manufacturers instructions. Recombinant His-tagged protein was eluted with elution buffer (50mM Tris, 750 mM NaCl, 500mM Imidazole, pH 7.5) and was collected in 1 ml fractions. Collected fractions were analysed for purified protein by SDS-PAGE. Fractions containing purified protein were dialysed in PBS to remove imidazole and purified protein was stored at - 80°C.

### DNA vaccine construction and preparation

Genes encoding selected proteins were sub-cloned from pCR^{®}T7/NT-TOPO into the pVAX1 DNA vaccine vector using the restriction enzymes BamH1 and EcoR1 and DNA ligation using DNA rapid ligation kit (Roche Diagnostics Ltd Lewes, UK). *E. coli* TOP10F' cells were transformed with recombinant pVAX1 vectors according to manufacturers instructions, and plasmids were purified from culture using a Qiagen Endofree Mega Prep kit (Qiagen, Crawley, UK). The correct sequence of the genes in the vector was determined by sequencing at MWG biotech (Milton Keynes, UK). DNA vaccine constructs were stored at -20°C.

### Immunisation and challenge with B. pseudomallei K96243

DNA vaccine constructs and purified proteins were produced for evaluation as candidate vaccines against *B. pseudomallei.* The concentrations of the proteins were ascertained by BCA assay (Pierce Biotechnologies) and purity was evaluated by SDS-PAGE and staining with PhastGel Blue R. Proteins were prepared for immunisation mixed 1:1 with MPL+TDM adjuvant (Sigma-Aldrich Co. Ltd., Poole, UK) and as 100 µg ml⁻¹ concentrations. DNA vaccine immunisations contained 100 µg of plasmid DNA in 0.25% (w/v) bupivicane hydrochloride (Astra pharmaceuticals, Kings Langley, UK) in 100 µl PBS. Groups of 6 female BALB/c mice (Charles River Laboratories Margate, UK) (5 to 6 weeks old) were used. Proteins were delivered by intraperitoneal (i.p.) injection of 100 µl of each protein in MPL+TDM® adjuvanton days 0, 14 and 28. DNA vaccines were injected intramuscularly (i.m.) with 50 µl of inoculum in each hind leg of each mouse on days 0 and 14. On day 28, mice immunised with DNA vaccines were boosted with a single i.p. immunisation of the corresponding purified protein at 100 µg ml⁻¹ concentrations. Blood was taken via the tail vein from each mouse on day 56. Blood samples were allowed to clot for 2-24 h at 4°C and the centrifuged for 10 min at 13,000 rpm and the serum was stored at - 20°C until use. On day 70 the mice were challenged with approximately 4 x 10⁴ colony forming units (cfu) of *B. pseudomallei* K96243 via the i.p. route. Groups of age matched mice were left untreated, or given adjuvant only or PVAX1 only as controls as controls.

### Antibody response analysis

The LolC-specific, OppA-specific and PotF-specific IgG1 or IgG2a responses in blood samples removed from mice prior to challenge were determined by enzyme-linked immunosorbant assay. Briefly, microtitre plates were coated with 5 µg ml⁻¹ of recombinant protein in PBS over night. Three columns on each plate were coated with anti-IgG1 (fab) or anti-IgG2a (fab) in order to produce a standard curve for quantification of IgG1 or IgG2a concentration respectively. The plates were blocked using 2% (w/v) bovine serum albumin in PBS (BSA-PBS) for 1 h at 37°C. Plates were washed and serum samples diluted in BSA-PBS were serially diluted 1:2 down the plate in duplicate and incubated for 2 h at 37°C. IgG1 or IgG2a isotype standards were also serially diluted 1:2 down the plate wells coated with anti-IgG1 (fab) or anti-IgG2a (fab) respectively. Three washes with PBS plus 0.05% (v/v) Tween 20 were carried out between each step. Goat anti-mouse IgG1 or IgG2a horseradish peroxidase conjugates diluted in BSA-PBS were added and after a further 2 h at 37 °C, bound antibody was visualised by adding ABTS substrate. The plates were incubated at room temperature for 20 min before measuring OD₄₁₄ₙₘ. Serum antibody concentration was calculated in ng ml⁻¹ using the standard curve (Ascent^{™} software version 2.4.2 Thermo Electron Corporation, Basingstoke, UK).

### Results

### Proteins selected as potential vaccine antigens

Genes encoding predicted ABC system proteins in the *B. pseudomallei* K96243 genome sequence were identified using bioinformatic tools. Three protein components of three different predicted ABC systems were selected for further investigation as potential antigens through their predicted roles in virulence or immunogenicity of *B. pseudomallei.*

OppA (BPSS2141) is a putative periplasmic binding protein component of the oligopeptide (Opp) ABC transporter system. In *E. coli,* OppA handles peptides that range widely in number of amino acids in length. The OppA protein is thought to be involved in the intracellular survival of *Listeria monocytogenes* in the macrophage and for bacterial growth in mice. This effect is probably due to either the uptake of peptides which are protective against the actions of the phagosome, or the uptake of peptides that bring about a modulation in the expression of proteins, thereby affording greater protection.

PotF (BPSL1555) is a putative periplasmic binding protein of a polyamine uptake system. In *E. coli,* PotF is a periplasmic binding protein of a putrescine-specific ABC transporter system.. The polyamines putrescine, spermidine and spermine are required for cell growth in eukaryotes and prokaryotes. PotF may have inhibitory effects upon *potFGHI* operon expression since PotD, a homologue of PotF, is thought to have an inhibitory effect on *potABCD* operon expression. As cellular accumulation of polyamines rises, a precursor of PotD acts as a transcriptional regulator, reducing the expression of polyamine uptake systems. The periplasmic location of PotF indicates that it may be presented to the host immune system and in this context may constitute an antigen.

LolC (BPSL2277) is a putative inner membrane protein of the LolCDE ABC transporter. In *E. coli* this system provides the energy for the transport of lipoproteins directed to the outer membrane, which are released from the inner membrane in complex with LolA, a periplasmic chaperone. Lipoproteins are synthesised in the cytoplasm of a bacterial cell and are exported to either the inner or the outer membrane for incorporation. The LolCDE complex is an essential ABC transporter for *E. coli* and the sole apparatus mediating the release of outer membrane lipoproteins from the inner membrane.

### Genes encoding selected proteins are detected in a range of Burkholderia strains

The genomic DNA of 11 different strains of *Burkholderia* sp. was screened by PCR for the presence of the genes encoding the selected proteins using oligonucleotide primer pairs designed to amplify short internal fragments of the genes. OppA, PotF and LoIC were found to be present in strains of *B. pseudomallei, B. thailandensis* and *B. mallei*. This evidence suggests that any future vaccine or therapeutic based on these proteins is likely to be protective against all strains of *Burkholderia* sp.

### Production of purified recombinant OppA, PotF and LolC proteins

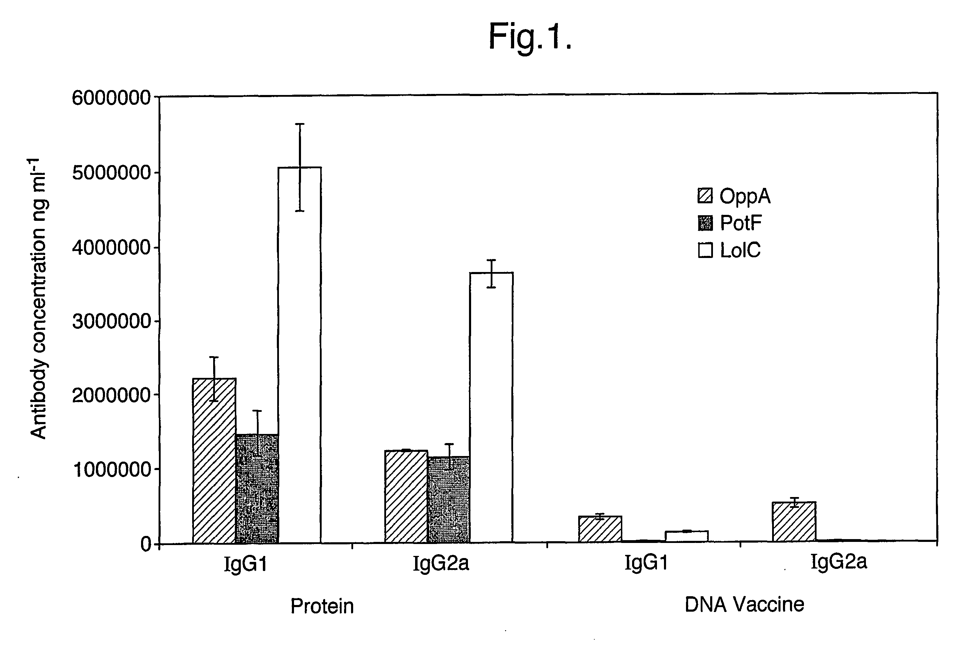

The predicted non-membrane/non-signal peptide regions of the selected proteins were identified using TMHMM v 2.0 (http://www.cbs.dtu.dk/services/TMHMM-2.0/) and SignalP (http://www.cbs.dtu.dk/services/SignalP/). *B. pseudomallei* K96243 DNA encoding the non-membrane/non-signal peptide regions of the selected proteins (figure 1a, b and c) was amplified by PCR and cloned into the expression vector pCR^{®}T7/NT-TOPO. This vector was used to allow expression of the proteins as fusions to a His-tag, enabling purification by His-Trap^{™}. *E. coli* BL21 (DE3) pLysS cells transformed with recombinant plasmids were induced to express the selected proteins which were subsequently purified by Immobilised Metal Affinity Chromatography. Following SDS-PAGE analysis, proteins of approximately 57 KDa, 38 KDa, and 24.5 KDa could be visualised (not shown) corresponding to the predicted molecular masses of OppA, PotF and LolC respectively. The purified proteins all reacted with anti-His monoclonal antibody in a Western blot (data not shown).

### Construction of DNA vaccines

To further investigate the potential of the selected proteins as vaccine antigens, DNA vaccines encoding the selected proteins were produced as an alternate vaccine delivery technology. The DNA vaccines were constructed by sub-cloning the genes encoding non-membrane/non-signal peptide regions of OppA, LolC and PotF from pCR^{®}T7/NT-TOPO into a pVAX1 vector without the inclusion of a His-Tag. The insertion of the genes into the pVAX1 vector was confirmed by restriction digest analysis and sequencing.

### Immunisation of mice with OppA, PotF and LolC

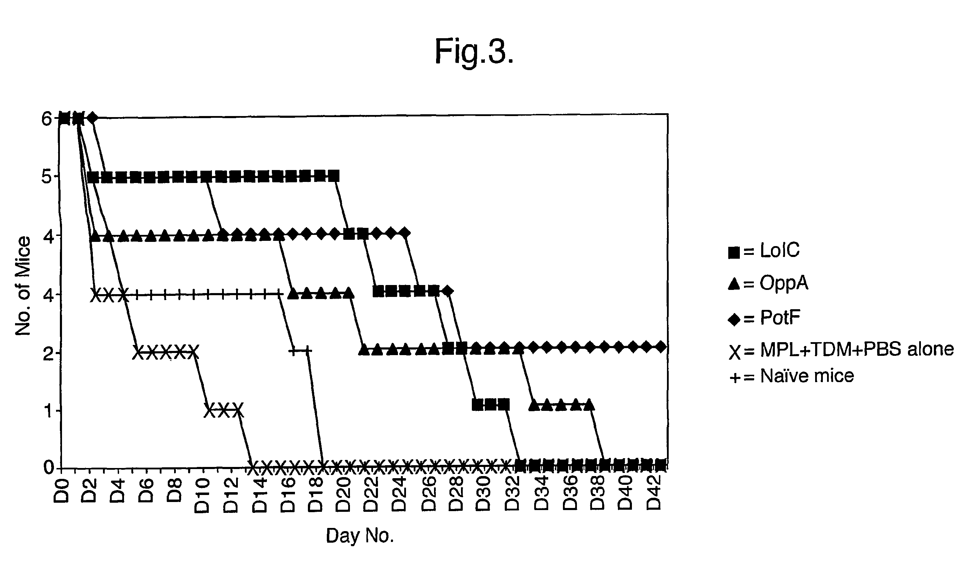

The non-membrane/non-signal peptide protein antigens, OppA, PotF and LolC and the corresponding DNA vaccines were used to immunise groups of mice. Briefly, mice were either administered with 3 doses of recombinant protein or 2 doses of DNA vaccine followed by 1 dose of recombinant protein (DNA prime/protein boost). Sera were collected via the tail vein 28 days after the final immunisation, and the sera from each group were pooled. The sera were analysed for specific antibody responses to each protein by ELISA (figure 3). Specific IgG1 and IgG2a responses to OppA, PotF and LolC could be detected in mice given the recombinant proteins. In comparison, only low levels of antibody could be detected in mice given the OppA and LolC DNA vaccines and mice given the PotF DNA vaccine had undetectable antibody responses. The recombinant proteins induced similar levels of IgG1 and IgG2a with a slight bias towards an IgG1 antibody (Th2 type) response. Mice immunised with PotF and OppA protein antigens showed greater IgG1 and IgG2a responses to the antigens in comparison to mice given the naïve control and adjuvant only controls. Mice immunised with LolC protein antigen produced greater IgG1 and IgG2a response than both the control mice and those immunised with PotF and OppA. The sera analysis indicates that the protein antigens, especially LolC, are potent immunogens, particularly in respect to IgG antibody response. DNA vaccines appear to be less effective at initiating an IgG antibody response. This is not unexpected, since it is known that DNA vaccines are effective stimulators of cellular immune responses.

### Protection afforded against B. pseudomallei

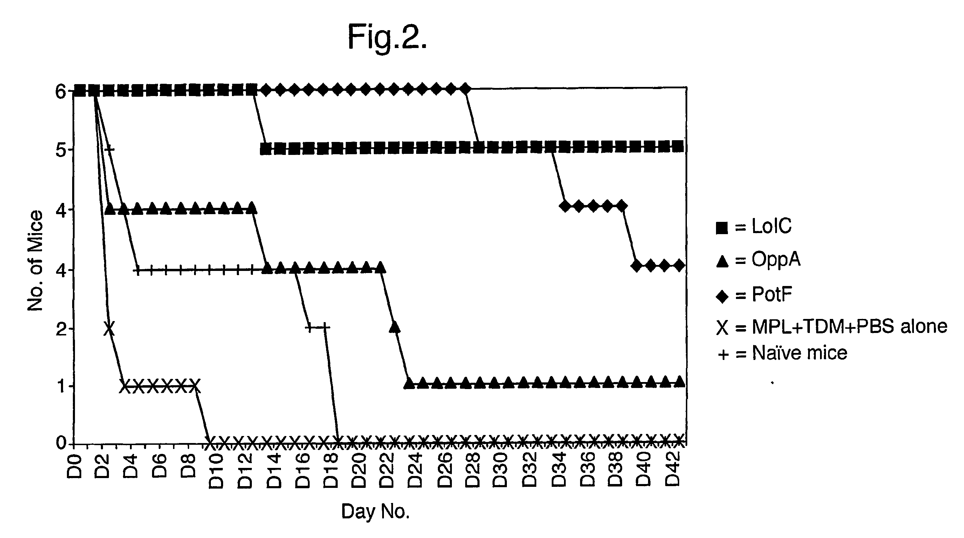

The mice immunised with recombinant OppA, PotF or LolC, or given corresponding DNA prime/protein boost immunisations were challenged with approximately 4x10⁴ cfu *B. pseudomallei* K96243 (equivalent to approximately 40 MLD) via the i.p. route and the health status of the mice was monitored for 42 days. Control mice given adjuvant or pVAX1 DNA only, or left untreated, all died by day 19 post-infection. However, protection afforded against *B. pseudomallei* infection could be observed in mice given either the proteins (figure 1) or DNA prime/protein boost (figure 2). Mice immunised with OppA protein demonstrated an extended time to death in comparison to both the naive control (p = <0.0001) and adjuvant only control mice (p = <0.0001) and also included 1/6 mouse that survived to the end of the experiment (figure 2). An extended time to death was also observed in the group of mice immunised with the PotF protein in comparison to both the naive control (p = <0.0001) and adjuvant only control mice (p = <0.0001), and 3/6 mice from this group survived to the end of the experiment. Immunisation with LolC protein had the greatest effect on survival, where 5/6 mice from this group survived to the end of the experiment. In comparison, mice immunised with DNA vaccines and given a protein boost, were not as well protected as those that received protein antigens. However, all the immunised mice demonstrated a significant extended time to death when compared to both the naive control (*p* = <0.0001) and adjuvant only control mice (p = <0.0001). In addition, of the mice immunised with PotF DNA vaccine prime/boost, 2/6 survived to the end of the experiment. The results of this study indicate that the selected protein antigens, delivered either as purified protein in adjuvant or in a DNA vaccine prime/protein boost regime may constitute protective antigens against *B. pseudomallei* infection. Since the highest level of protection afforded was by the LolC purified recombinant protein it is possible that IgG antibody responses are important in protection. The high IgG concentrations present may prevent colonisation by *B. pseudomallei* either through the process of opsonisation or direct neutralisation of the pathogen. However, an extended time to death was observed in all DNA vaccine groups and 2 mice immunised with the DNA vaccine encoding PotF survived the experiment. Since the DNA vaccines were ineffective at inducing IgG responses, this indicates that protection was mediated through an alternative immune mechanism. Further work will be required understand the mechanisms of protection afforded.

The results described herein identify OppA, PotF and LolC proteins of *B. pseudomallei* as protective antigens. This is the first known demonstration of any protein providing protection against *B. pseudomallei* infection. Combinations of these proteins could be used together to enhance the protection observed with individual proteins. Since the genes encoding the protective proteins have been detected in a range of *B. pseudomallei* strains, the antigens should be protective against all *B. pseudomallei* strains. Also, as the genes have been detected in a *B. mallei* strain, the proteins may also protect against glanders, the disease resulting from *B. mallei* infection. Therefore, these proteins should cross-protect against both melioidosis and glanders. Furthermore, since homologues of the protective proteins can be found in a range of bacteria, OppA, PotF and LolC may provide protection against a range of bacterial infections and as a result could be used as a generic vaccine. As genes encoding the protective proteins are not present in human or mouse genomes it is unlikely that a vaccine based on these proteins would have a deleterious affect. Since it has also been observed that antibody response maybe important in protection, it is possible that anti-sera raised against proteins could be used therapeutically, with the potential as use in generic therapy.

### Sequence Listings:

**SEQ ID NO 1: Nucleotide sequence ("PotF")** 
ATGAAGGATACGCAGCTCAACGTCTATAACTGGTCGGACTACATTGCGAAGGACACGATC
CCGAACTTCGAGAAGCAGACGGGCGTCAAGGTCCGCTACGACAACTACGACAGCGACGAC
ACGCTGCAGGCGAAGCTTTTGACCGGCAACTCGGGCTACGACATCGTCGTGCCGACGAGC
AACTACGCGGGCAAGCAGATCCAGGCCGGCATCTTCACGCCGCTCGACAAGTCGAAGCTG
CCGAACCTCAAGTATCTCGATGCGCAACTGATGGCGCTCGTCGCGGGCGCCGATCCGGGC
AACAAGTACGTGGTGCCCTGGGCGTATGGCACGACGGGCCTCGGCTACAACGTCGACAAG
GCGCAGAAGGTGCTCGGCAAGGTGCCGCTCGACAACTGGGACATCCTGTTCAAGCCGGAG
AACCTCTCGAAGCTGAAGACGTGCGGCGTGTCGGTGCTCGACGCGCCGGACCAGATGTTC
GCGGCGACGCTGCACTACATCGGCAAGGATCCGATGAGCACGAACCCGGCCGACTACCAG
GCCGCGATGCAGGTGCTCAAGAAGATCCGCCCGTACATCACGCAGTTCAACTCGTCGGGC
TACATCAACGACATGGTCGGCGGCGACATCTGCTTCGCGTTCGGCTGGTCGGGCGACGTC
GTGATCGCGAAGCACCGCGCGCTCGAGGCGAAGAAGCCGTACAAGCTCGAGTACTACGTG
CCGAAGGGCGGCGCGCCCGTATGGTTCGACGTGATGGCGATCCCGAAGGACGCGAAGAAC
AAGGACGCCGCGCTTCAATGGATCAACTACATCGAGGATCCGAAGGTTCACGCGTCGATC
ACGAACGCGGTCTACTACCCGAGCGCGAACGCGCAAGCGCGCAAGTACGTGCGCCCGGAT
GTCGCGAACGACCCGGCCGTGTACCCGCCTCCGGACGTCGTGAAGACGCTGTTCCTGCTC
AAGCCGCTGCCGCCCGAGATCCAGCGCCTGCAGACGCGTCTTTGGACCGAGCTGAAATCG
GGACGCTGA
**SEQ ID NO 2 Nucleotide sequence ("OppA")** 
ATGTCGAATGTCACGCTCGCGGATCAGCAGGACCTCACGCGGCAGGTGCCCGCGGAGGTC
GAATCGCTCGACCCCGCGCACATCGAATCGTGGACCGGCAACACGATCGGCCTGGATCTG
TTCGAGGGGCTCGCGCGCATCGACGCGAGCGGGGCGGTGGTGCCGGGCGTCGCGCAGGCG
TGGGAGCACAAGGCGCCGGATACGTGGATCTTCAAGCTGCGCCGCGACGCGAAGTGGAGC
AACGGGCAGCCCGTGACGGCCGCGGATTTCGTATACGCGTGGCAGCGTCTCGCCGATCCG
AAGACGGGTTCGAAATACACGATCCTCGTCGAGTTCGTGAAGAACGCCTCGGCGATCATC
GCGGGCAAGCAGCCGCCCGGCGATCTCGGCATTCGCGCGATCGATCCGTACACGATCGAG
GTGAAGACCGAGGTGCCCGTGTCGTATTTCCCCGAGCTCACCGCGATGGCGCCGCTCACC
CCCGTGAACAAGGACGCGGTCGCGAAGTTCGGCGACGCATGGACGCGCCCGAAGAACATC
GTGAGCAACGGCCCTTACACGCTCGTCGACTGGCAGCCGAACAACCGGATCGTGATGGCG
AAGAGCGACAAGTACTGGAACGCGCGCAACGTCGTGATCCGCAAGGTGACGTACCTGCCG
ATCGAGAACGACGAGACCGCGCTGCGGATGTACCAGGCGGGCCAGATCGACTACACGTAT
TCGATTCCCGCGGGCGGCTTCGGCCAGATCAGCAAGCAGTTCGGCAAGGAGCTGCGCCCG
GGGCTGCAGCTCGCGACGTATTACTACCTGAAGAACAGCGATCCGGCGCTCAAGGAC
AAGCGCGTGCGCGAAGCGCTCGCGATGGTGCTCGACCGCGAGATCCTCACGTCGAAGATC
ACGCAGGCGGGCGAAGTGCCGATGTACGGCTGATGCCGAAGGGCGTGAAGGGCGTGCAG
CGGCCGTTCACGCCGGACTGGGCGTCGTGGCCGATGGCGAGGCGCGTCGACTACGCGAAG
AACCTGCTGAAGCAGGCGGGCCATGGCGACGCGAATCCGCTCACGTTCACGCTGACCTAC
AACACGAACGACCTGCACAAGAAGGTCGCGCTGTTCGCGGCGTCCGAATGGCGCACGAAG
CTCGGCGTGACGGCGAAGCTCGAGAACGTCGAGTTCAAGGTGCTGATGAAGCAGCGCCAC
GACGGCAAGGTGCAGATCGCGCGCGACGGCTGGTTCGCCGACTACAACGACGCGATGACG
TTCTTCGACCTGATCCGCTGCGGCAGCTCGCAGAACACGGTCGGCTACTGCAACCCGAAG
GTCGATTCGCTCGTCGCCGAGGCGAACCAGAAGCTCGATGACGGCGCGCGCGCGGCGCTG
CTCACGCAGGCGCACGATCTCGCGATGAACGACTATCCGATGGTGCCGCTCTTCCAGTAT
TCGGCGGACCGCCTCGTGAAGTCGTACGTCGGCGGCTACACGCTGACGAACTACATCGAC
ATGCGCGCCTCGCAGGACATGTACCTGATCAAGCACTGA
**SEQ ID NO 3 Nucleotide sequence ("LolC")** 
ATGGTGCTGTCGGTGATGAACGGCTTCCAGAAAGAGGTGCGCGACCGGATGCTGTCGGTG
CTCGCGCACGTCGAGATCTTCTCGCCGACGGGCTCGATGCCCGACTGGCAACTGACCGCG
AAGGAAGCGCGCCTGAACCGCTCGGTGATCGGCGCGGCGCCGTATGTCGACGCGCAGGCG
CTGCTCACGCGGCAGGACGCGGTGAGCGGCGTGATGCTGCGCGGCGTCGAGCCGTCGCTC
GAGCCGCAGGTGTCCGACATCGGCAAGGACATGAAGGCGGGCGCGCTCACCGCGCTCGCG
CCCGGCCAGTTCGGGATCGTGCTCGGCAACGCGCTCGCGGGCAATCTCGGCGTGGGCGTC
GGCGACAAGGTCACGCTCGTCGCGCCGGAAGGCACGATCACGCCGGCCGGGATGATGCCG
CGCCTGAAGCAGTTCACGGTGGTCGGCATCTTCGAGTCCGGGCACTACGAATACGACAGC
ACGCTTGCGATGATCGACATCCAGGATGCGCAGGCGCTCTTCAGGCTGCCCGCGCCGACG
GGCGTGCGGCTGCGCCTCACGGACATGCAGAAGGCGCCGCAGGTCGCGCGCGAGCTCGCG
CACACGCTGTCGGGCGACCTGTACATCCGCGACTGGACGCAGCAGAACAAGACGTGGTTC
TCCGCGGTGCAGATCGAGAAGCGGTGA
**SEQ ID NO 4: Amino Acid ("LolC")** 
VLSVMNGFQKEVRDRMLSVLAHVEIFSPTGSMPDWQLTAKEARLNRSVIGAAPYVDAQAL
LTRQDAVSGVMLRGVEPSLEPQVSDIGKDMKAGALTALAPGQFGIVLGNALAGNLGVGVG
DKVTLVAPEGTITPAGMMPRLKQFTVVGIFESGHYEYDSTLAMIDIQDAQALFRLPAPTG
VRLRLTDMQKAPQVARELAHTLSGDLYIRDWTQQNKTWFSAVQIEKR
**SEQ ID NO 5: Amino Acid ("PotF")** 
KDTQLNVYNWSDYIAKDTIPNFEKQTGVKVRYDNYDSDDTLQAKLLTGNSGYDIVVPTSN
YAGKQIQAGIFTPLDKSKLPNLKYLDAQLMALVAGADPGNKYVVPWAYGTTGLGYNVDKA
QKVLGKVPLDNWDILFKPENLSKLKTCGVSVLDAPDQMFAATLHYIGKDPMSTNPADYQA
AMQVLKKIRPYITQFNSSGYINDMVGGDICFAFGWSGDVVIAKHRALEAKKPYKLEYYVP
KGGAPVWFDVMAIPKDAKNKDAALQWINYIEDPKVHASITNAVYYPSANAQARKYVRPDV
ANDPAVYPPPDVVKTLFLLKPLPPEIQRLQTRLWTELKSGR
**SEQ ID NO 6: Amino Acid ("OppA")**

SNVTLADQQDLTRQVPAEVESLDPAHIESWTGNTIGLDLFEGLARIDASGAVVPGVAQAW
EHKAPDTWIFKLRRDAKWSNGQPVTAADFVYAWQRLADPKTGSKYTILVEFVKNASAIIA
GKQPPGDLGIRAIDPYTIEVKTEVPVSYFPELTAMAPLTPVNKDAVAKFGDAWTRPKNIV
SNGPYTLVDWQPNNRIVMAKSDKYWNARNVVIRKVTYLPIENDETALRMYQAGQIDYTYS
IPAGGFGQISKQFGKELRPGLQLATYYYYLKNSDPALKDKRVREALAMVLDREILTSKIT
QAGEVPMYGLMPKGVKGVQRPFTPDWASWPMARRVDYAKNLLKQAGHGDANPLTFTLTYN
TNDLHKKVALFAASEWRTKLGVTAKLENVEFKVLMKQRHDGKVQIARDGWFADYNDAMTF
FDLIRCGSSQNTVGYCNPKVDSLVAEANQKLDDGARAALLTQAHDLAMNDYPMVPLFQYS
ADRLVKSYVGGYTLTNYIDMRASQDMYLIKH

## Claims

1. A protein expressed by an ABC system gene of *Burkholderia pseudomallei,* selected from the group consisting of OppA, PotF and LolC, or a fragment or a variant of said protein, wherein the protein, fragment, or variant is capable of producing a protective immune response against *Burkholderia mallei* or *Burkholderia pseudomallei*, for use as a medicament.

2. A protein according to claim 1 which is a polypeptide according to SEQ ID NO.4, SEQ ID NO.5 or SEQ ID NO.6 or a fragment or a variant of any of these wherein the protein, fragment or variant is capable of producing a protective immune response against *B.pseudomallei* or *B.mallei*

3. A pharmaceutical composition comprising a protein expressed by an ABC system gene of Burkholderia pseudomallei, selected from the group consisting of OppA, PotF and LolC, or a fragment or a variant of said protein, wherein the protein, fragment, or variant is capable of producing a protective immune response against *Burkholderia mallei* or *Burkholderia pseudomallei,* in combination with a pharmaceutically acceptable carrier or excipient.

4. A pharmaceutical composition according to claim 3 wherein the protein, fragment or variant is selected from the group comprising proteins encoded by a nucleic acid of SEQ ID NO1, SEQ ID NO 2 and SEQ ID NO 3.

5. A pharmaceutical composition according to claim 3 in which the protein comprises SEQ. ID NO.4, SEQ ID NO.5 or SEQ ID NO.6 or protective fragments or protective variants of any of these.

6. A pharmaceutical composition according to any of claims 3 to 5 which further comprises an adjuvant.

7. A nucleic acid which encodes a protein according to either of claims 1 or 2 for use as a medicament.

8. A pharmaceutical composition comprising a nucleic acid which encodes a protein according to either of claims 1 or 2.

9. A pharmaceutical composition according to claim 8 which comprises SEQ ID NO 1, 2 or 3, or a fragment or a variant thereof

10. Use of a protein according to either of claims 1 or 2 in the manufacture of a medicament for the treatment of *Burkholderia* infection.

11. Use of a protein according to claim 10 wherein the *Burkholderia* infection is caused by *Burkholderia mallei* or *Burkholderia pseudomallei* or *Burkholderia thailandensis.*

## Patentansprüche

1. Protein, das von einem Gen für das ABC-System von *Burkholderia pseudomallei* exprimiert wird und aus der Gruppe ausgewählt ist, die besteht aus OppA, PotF und LolC, oder ein Fragment oder eine Variante dieses Proteins, wobei das Protein, das Fragment oder die Variante befähigt ist, eine schützende Immunantwort gegen *Burkholderia mallei* oder *Burkholderia pseudomallei* zu erzeugen, zur Verwendung als Arzneimittel.

2. Protein nach Anspruch 1, bei dem es sich um ein Polypeptid gemäß SEQ ID NO:4, SEQ ID NO:5 oder SEQ ID NO:6 oder um ein Fragment oder eine Variante dieser Polypeptide handelt, wobei das Protein, das Fragment oder die Variante befähigt ist, eine schützende Immunantwort gegen *B. pseudomallei* oder *B. mallei* zu erzeugen.

3. Pharmazeutische Zusammensetzung, die enthält: ein Protein, das von einem Gen für das ABC-System von *Burkholderia pseudomallei* exprimiert wird und aus der Gruppe ausgewählt ist, die besteht aus OppA, PotF und LolC, oder ein Fragment oder eine Variante dieses Proteins, wobei das Protein, das Fragment oder die Variante befähigt ist, eine schützende Immunantwort gegen *Burkholderia mallei* oder *Burkholderia pseudomallei* zu erzeugen, in Kombination mit einem pharmazeutisch akzeptablen Träger oder Excipiens.

4. Pharmazeutische Zusammensetzung nach Anspruch 3, bei der das Protein, das Fragment oder die Variante aus der Gruppe ausgewählt ist, die Proteine umfasst, die durch eine Nucleinsäure gemäß SEQ ID NO:1, SEQ ID NO:2 und SEQ ID NO:3 codiert werden.

5. Pharmazeutische Zusammensetzung nach Anspruch 3, bei der es sich bei dem Protein um ein Protein mit der Sequenz SEQ ID NO:4, SEQ ID NO:5 oder SEQ ID NO:6 oder schützende Fragmente oder schützende Varianten dieser Proteine handelt.

6. Pharmazeutische Zusammensetzung nach einem der Ansprüche 3 bis 5, die ferner ein Adjuvans enthält.

7. Nucleinsäure, die ein Protein nach Anspruch 1 oder 2 codiert, zur Verwendung als Arzneimittel.

8. Pharmazeutische Zusammensetzung, die eine Nucleinsäure enthält, die ein Protein nach Anspruch 1 oder 2 codiert.

9. Pharmazeutische Zusammensetzung nach Anspruch 8, die eine Nucleinsäure gemäß SEQ ID NO:1, SEQ ID NO:2 oder SEQ ID NO:3 oder ein Fragment oder eine Variante davon enthält.

10. Verwendung eines Proteins nach Anspruch 1 oder 2 bei der Herstellung eines Arzneimittels zur Behandlung von *Burkholderia*-Infektionen.

11. Verwendung eines Proteins nach Anspruch 10, wobei die *Burkholderia*-Infektion durch *Burkholderia mallei* oder *Burkholderia pseudomallei* oder *Burkholderia thailandensis* hervorgerufen ist.

## Revendications

1. Protéine exprimée par un gène du système ABC de *Burkholderia pseudomallei,* choisie dans le groupe constitué par OppA, PotF et LolC, ou un fragment ou un variant de ladite protéine, où la protéine, le fragment ou le variant est capable de produire une réponse immunitaire protectrice contre *Burkholderia mallei* ou *Burkholderia pseudomallei,* pour une utilisation en tant que médicament.

2. Protéine selon la revendication 1, qui est un polypeptide selon SEQ ID NO : 4, SEQ ID NO : 5 ou SEQ ID NO : 6 ou un fragment ou un variant de l'une quelconque de celles-ci où la protéine, le fragment ou le variant est capable de produire une réponse immunitaire protectrice contre *B. pseudomallei* ou *B. mallei.*

3. Composition pharmaceutique comprenant une protéine exprimée par un gène du système ABC de *Burkholderia pseudomallei,* choisie dans le groupe constitué par OppA, PotF et LolC, ou un fragment ou un variant de ladite protéine, où la protéine, le fragment ou le variant est capable de produire une réponse immunitaire protectrice contre *Burkholderia mallei* ou *Burkholderia pseudomallei,* en combinaison avec un support ou un excipient pharmaceutiquement acceptable.

4. Composition pharmaceutique selon la revendication 3, dans laquelle la protéine, le fragment ou le variant est choisi dans le groupe comprenant des protéines codées par un acide nucléique de SEQ ID NO : 1, SEQ ID NO : 2 et SEQ ID NO : 3.

5. Composition pharmaceutique selon la revendication 3, dans laquelle la protéine comprend SEQ ID NO : 4, SEQ ID NO : 5 ou SEQ ID NO : 6 ou des fragments protecteurs ou des variants protecteurs de l'une quelconque de celles-ci.

6. Composition pharmaceutique selon l'une quelconque des revendications 3 à 5, qui comprend en outre un adjuvant.

7. Acide nucléique qui code pour une protéine selon l'une ou l'autre des revendications 1 ou 2 pour une utilisation en tant que médicament.

8. Composition pharmaceutique comprenant un acide nucléique qui code pour une protéine selon l'une ou l'autre des revendications 1 ou 2.

9. Composition pharmaceutique selon la revendication 8, qui comprend SEQ ID NO : 1, 2 ou 3, ou un fragment ou un variant de celles-ci.

10. Utilisation d'une protéine selon l'une ou l'autre des revendications 1 ou 2, dans la fabrication d'un médicament destiné au traitement d'une infection par *Burkholderia.*

11. Utilisation d'une protéine selon la revendication 10, où l'infection par *Burkholderia* est provoquée par *Burkholderia mallei* ou *Burkholderia pseudomallei* ou *Burkholderia thailandensis.*
